# EUROPÄISCHE PATENTSCHRIFT

(11) **EP 2 916 894 B1**
(45) Veröffentlichungstag und Bekanntmachung des Hinweises auf die Patenterteilung: **08.05.2019**
(21) Anmeldenummer: 13788760.0
(22) Anmeldetag: 08.11.2013
(51) Int. Cl.: A61M 5/32, A61M 5/34, A61M 5/31, A61M 5/50

(54) **SPRITZENVERSCHLUSS**
SYRINGE CLOSURE
OBTURATEUR DE SERINGUE

(30) Priorität: 09.11.2012 DE 102012022008
(43) Veröffentlichungstag der Anmeldung: 16.09.2015
(73) Patentinhaber: Vetter Pharma-Fertigung GmbH & Co. KG, 88212 Ravensburg (DE)
(72) Erfinder: GLOCKER, Joachim, 88250 Weingarten (DE)
(74) Vertreter: Kordel, Mattias
(86) Internationale Anmeldenummer: PCT/EP2013/073320
(87) Internationale Veröffentlichungsnummer: WO 2014/072440

(56) Entgegenhaltungen:
- EP-A1- 1 647 294
- EP-A1- 1 693 079
- WO-A1-2006/087763
- WO-A1-2012/116792
- WO-A2-01/74424
- WO-A2-2004/052432
- DE-A1- 19 956 243
- DE-A1-102011 015 112

## Beschreibung

Die Erfindung betrifft einen Spritzenverschluss für eine Spritze gemäß Oberbegriff des Anspruchs 1.

Spritzenverschlüsse der hier angesprochenen Art sind bekannt und beschrieben, siehe zum Beispiel den Verschluss in der WO 01074424 oder den Verschluss in der DE 199 56 243. In der Regel sind sie als Originalitätsverschluss ausgebildet, nämlich derart, dass unberechtigte Manipulationen am Verschluss für einen Benutzer ohne Weiteres erkennbar sind. Der Spritzenverschluss wird auf einen von dem Grundkörper der Spritze ausgehenden endständigen Fortsatz aufgedrückt, der in der Regel konisch ausgebildet ist. An dem Fortsatz ist in einem Abstand zu seinem freien Ende mindestens eine Vertiefung vorgesehen, die vorzugsweise ringförmig ausgebildet ist. Nach dem Aufsetzen des Spritzenverschlusses schnappt dieser mit seinem im Bereich der Vertiefung liegenden Rand in diese ein und wird so am Fortsatz der Spritze gehalten. Um einen sicheren Verschluss der Spritze zu gewährleisten, soll der Spritzenverschluss sich von dem Fortsatz nicht abziehen lassen. Auch soll eine Relativdrehung zwischen Spritze und Verschluss verhindert werden. Daher ist es erforderlich, eine starke Klemmkraft aufzubauen. Um dies zu gewährleisten, ist vorgesehen, dass der Spritzenverschluss im Bereich der Vertiefung sicher anliegt, wobei der Außendurchmesser im Bereich der Vertiefung kleiner ist als in dem unmittelbar daran angrenzenden Bereich des Fortsatzes. Bei bekannten Spritzenverschlüssen ist vorgesehen, dass diese beim Aufsetzen auf den Fortsatz aufgedehnt werden, sodass sie durch Zurückschnappen in die Vertiefung sicheren Halt am Fortsatz der Spritze finden.

Es hat sich herausgestellt, dass beim Aufsetzen des Spritzenverschlusses auf den Fortsatz relativ hohe Aufpresskräfte erforderlich sind, die zu einem Bruch der in der Regel aus Glas bestehenden Spritze führen können. Es hat sich auch gezeigt, dass das beim Aufsetzen überdehnte Material des Spritzenverschlusses Ermüdungserscheinungen zeigen kann, die einen sicheren Halt des Spritzenverschlusses an der Spritze verhindern.

Aufgabe der Erfindung ist es daher, einen Spritzenverschluss zu schaffen, der an einer Spritze sicheren Halt findet und die genannten Nachteile nicht aufweist.

Zur Lösung dieser Aufgabe wird ein Spritzenverschluss vorgeschlagen, der die in Anspruch 1 genannten Merkmale aufweist. Der Spritzenverschluss zeichnet sich durch eine Verriegelungseinrichtung mit mindestens einem Verriegelungselement aus, welches in einer ersten Funktionsstellung nach dem Aufsetzen des Spritzenverschlusses auf den Fortsatz einer Spritze eine Freigabeposition einnimmt. Mit dem Begriff "Freigabeposition" wird hier ausgedrückt, dass der Spritzenverschluss mehr oder weniger locker auf dem Fortsatz sitzt und dabei allenfalls sehr geringe Haltekräfte aufgebaut werden, die nicht ausreichen, den erforderlichen sicheren Halt des Spritzenverschlusses an der Spritze, besonders an deren Fortsatz zu gewährleisten. Beim ersten Aufsetzen des Spritzenverschlusses auf eine Spritze wird dieser, anders als bei bekannten Systemen, allenfalls nur sehr wenig aufgeweitet und nur leicht am Fortsatz gehalten.

Die Verriegelungseinrichtung ist so ausgelegt, dass deren mindestens ein Verriegelungselement in einer zweiten Funktionsstellung einen Formschluss mit der mindestens einen Vertiefung am Fortsatz gewährleistet, sodass der Spritzenverschluss verriegelnd am Fortsatz gehalten wird. In dieser zweiten Funktionsstellung wird der Spritzenverschluss mit der gewünschten endgültigen Haltekraft am Fortsatz gehalten und befindet sich daher in der Verriegelungsposition.

Besonders bevorzugt wird ein Ausführungsbeispiel des Spritzenverschlusses, welches sich dadurch auszeichnet, dass das mindestens eine Verriegelungselement mit dem Spritzenverschluss derart zusammenwirkt, dass Bereiche von diesem in die mindestens eine Vertiefung gedrängt werden. Diese Bereiche des Spritzenverschlusses sind also beim Aufsetzen auf den Fortsatz einer Spritze demnach nicht so angeordnet, dass sie mit der mindestens einen Vertiefung in Formschluss treten und Haltekräfte aufbauen. Vielmehr ist es eben möglich, dass diese Bereiche nach dem ersten Aufsetzen des Verschlusses auf den Fortsatz zur Erreichung der Verriegelungsposition durch das mindestens eine Verriegelungselement in die Vertiefung gedrängt werden. Es ist also möglich, den Spritzenverschluss ohne große Kräfte auf den Fortsatz einer Spritze aufzusetzen und erst nach dem Aufsetzen durch Einwirkung des mindestens einen Verriegelungselements Bereiche des Spritzenverschlusses in die mindestens eine Vertiefung zu drängen und damit einen sicheren Halt zu gewährleisten.

Bei einem besonders bevorzugten Ausführungsbeispiel ist vorgesehen, dass die Verriegelungseinrichtung eine Anzahl von Verriegelungselementen aufweist, die einem vorzugsweise ringförmig ausgebildeten Tragkörper zugeordnet und an diesem in vorzugsweise gleichem Umfangsabstand zueinander angeordnet sind. Dadurch, dass mehrere Verriegelungselemente in der Verriegelungsposition einen Formschluss mit einer Vertiefung am Fortsatz gewährleisten, kann ein besonders sicherer Halt des Spritzenverschlusses an der Spritze gewährleistet werden. Dabei kann vorgesehen werden, dass jedem der Verriegelungselemente eine eigene separate Vertiefung am Fortsatz der Spritze zugeordnet ist, oder dass wenigstens einige der Verriegelungselemente in eine gemeinsame Vertiefung eingreifen. Besonders bevorzugt wird eine ringförmige Vertiefung am Fortsatz vorgesehen, in welche alle Verriegelungselemente eingreifen.

Weitere Ausführungsbeispiele ergeben sich aus den Unteransprüchen.

Die Erfindung wird im Folgenden anhand der Zeichnung näher erläutert. Es zeigen:
- Figur 1: das Ende einer Spritze mit einem Spritzenverschluss im Längsschnitt, unmittelbar vor Aufsetzen des Verschlusses auf die Spritze;
- Figur 2: die Spritze und den Spritzenverschluss nach Figur 1 unmittelbar nach dem Aufsetzen des Spritzenverschlusses auf die Spritze in einer Freigabeposition;
- Figur 3: den auf die Spritze fest aufgesetzten Spritzenverschluss in Verriegelungsposition;
- Figur 4: den in den Figuren 1 bis 3 dargestellten Spritzenverschluss in Unteransicht und
- Figur 5: eine Verriegelungseinrichtung in perspektivischer Ansicht von schräg oben.

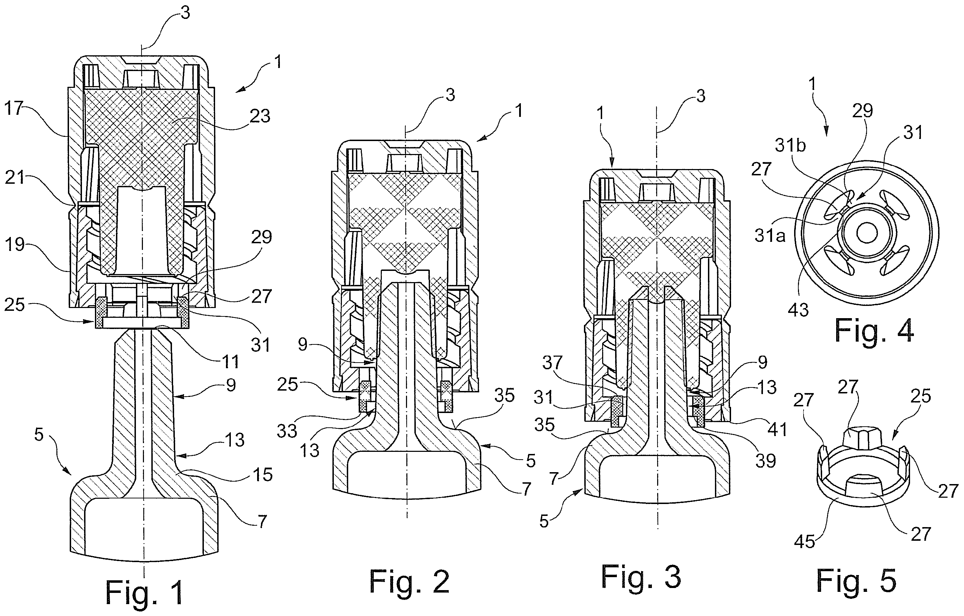

In Figur 1 ist ein Spritzenverschluss 1 im Längsschnitt dargestellt, wobei die Schnittebene so angeordnet ist, dass in dieser die Längsachse 3 des Spritzenverschlusses 1 liegt.

Der Spritzenverschluss 1 ist hier unmittelbar über einer Spritze 5 angeordnet, deren Mittelachse mit der Längsachse 3 zusammenfällt. Die Spritze weist einen Grundkörper 7, den Spritzenzylinder, auf und einen von diesem ausgehenden endständigen Fortsatz 9, der vorzugsweise konisch ausgebildet ist und ein freies Ende 11 aufweist. Bei konischer Ausgestaltung verjüngt sich der Fortsatz 9 ausgehend vom Grundkörper 7 der Spritze 5 in Richtung auf sein freies Ende 11.

In einem Abstand zu dem freien Ende 11 des Fortsatzes ist mindestens eine Vertiefung 13 vorgesehen, die hier nahe der Basis 15 des Fortsatzes 9 liegt, über welche dieser in den Grundkörper 7 übergeht.

Der Fortsatz 9 kann ein oder mehrere derartige Vertiefungen 13 aufweisen, vorzugsweise ist eine einzige ringförmige Vertiefung vorgesehen, in deren Bereich der Außendurchmesser des Grundkörpers 7 kleiner ist als in einem darüberliegenden Bereich. Die ringförmige Vertiefung liegt in einer gedachten Ebene, auf der die Längsachse 3 senkrecht steht.

Der Spritzenverschluss 1 kann als Sicherheits- oder Originalitätsverschluss aufgebaut sein, wie dies gerade im medizinischen Bereich üblich ist. Derartige Spritzenverschlüsse sind vorzugsweise zweiteilig ausgebildet, wobei ein oberer Bereich 17 mit einem unteren Bereich 19 über einen Sollbruchlinie 21 verbunden ist, die beim Öffnen des Spritzenverschlusses 1, also beim Abnehmen des oberen Bereichs 17, irreversibel verformt, insbesondere aufgerissen wird, sodass für einen Benutzer Manipulationen an dem Spritzenverschluss ohne Weiteres erkennbar sind. Derartige bekannte Spritzenverschlüsse 1 weisen in der Regel noch mindestens ein Dichtelement 23 auf, welches sich nach dem Aufsetzen des Spritzenverschlusses 1 von außen dichtend an die Umfangsfläche des Fortsatzes 9 anlegt und insbesondere dessen freies Ende 11 ebenfalls dichtend verschließt.

Der Spritzenverschluss 1 weist eine Verriegelungseinrichtung 25 auf, die mit mindestens einem Verriegelungselement 27 versehen ist. Bei dem hier dargestellten Ausführungsbeispiel greift das Verriegelungselement 27 von unten, also von der offenen, der Spritze 5 zugewandten Seite des Spritzenverschlusses 1 zumindest bereichsweise in diesen ein. Durch diese vorzugsweise vorgesehene Anordnung der Verriegelungseinrichtung 25 wird eine Montageeinheit zwischen Spritzenverschluss 1 und Verriegelungseinrichtung 25 geschaffen, sodass diese beiden Elemente gemeinsam gehandhabt und auf eine Spritze 5 aufgesetzt werden können.

Figur 1 ist zu entnehmen, dass das mindestens eine Verriegelungselement 27 in einer Ausnehmung 29 angeordnet ist, die radial innen liegend, das heißt, in Richtung auf die Längsachse 3 gesehen, von einem Wandbereich 31 begrenzt wird.

Figur 2 zeigt den Spritzenverschluss 1 und die Spritze 5 wiederum im Längsschnitt. Gleiche und funktionsgleiche Teile werden mit identischen Bezugszeichen versehen, sodass insofern auf die Beschreibung zu Figur 1 verwiesen wird, um Wiederholungen zu vermeiden.

Figur 2 zeigt, dass der Spritzenverschluss 1 gemeinsam mit der Verriegelungseinrichtung 25 auf den Fortsatz 9 aufgeschoben ist, wobei sich in dieser in Figur 2 wiedergegebenen Position noch keine derartigen Haltekräfte am Fortsatz 9 ergeben, dass der Spritzenverschluss 1 mit der für den Transport und die Lagerung erforderlichen Haltekräfte an der Spritze 5 gehalten wird. Die Verriegelungseinrichtung 25 ist in der hier wiedergegebenen Position des Spritzenverschlusses 1 nicht aktiviert, sodass sich das mindestens eine Verriegelungselement 27 in einer Freigabeposition befindet.

Die Verriegelungseinrichtung 25 befindet sich in einer gegenüber Figur 1 unveränderten Relativposition gegenüber dem Spritzenverschluss 1, wobei ein unterer Rand 33 der Verriegelungseinrichtung 25 sich in einem Abstand zu einer Schulter 35 des Grundkörpers 7 der Spritze 5 befindet.

Figur 3 zeigt den Spritzenverschluss 1 auf der Spritze 5 in einer zweiten Funktionsstellung, nämlich in Verriegelungsposition. Hier ist der Spritzenverschluss 1 gänzlich auf den Fortsatz 9 aufgeschoben, sodass dieser vorzugsweise rundum, insbesondere aber im Bereich seines freien Endes 11 von dem Dichtelement 23 eingeschlossen ist.

Die Position der mindestens einen Vertiefung 13 am Fortsatz 9 ist bei dem hier dargestellten Ausführungsbeispiel des Spritzenverschlusses 1 derart gewählt, dass bei einem vollständigen Aufdrücken des Spritzenverschlusses 1 auf die Spritze 5 die Schulter 35 des Grundkörpers 7 der Spritze 5 durch Anlage am unteren Rand 33 der Verriegelungseinrichtung 25 diese in den Spritzenverschluss 1 hineinverlagert. Mit anderen Worten: Der Spritzenverschluss 1 wird nach Anschlag des unteren Randes 3 an der Schulter 35 über die in Richtung der Längsachse 3 lagefest gehaltene Verriegelungseinrichtung 25 geschoben. Es findet also eine Relativbewegung zwischen dem Spritzenverschluss 1 und der Verriegelungseinrichtung 5 statt, während der Spritzenverschluss 1 in seine in Figur 3 wiedergegebene Endposition, der Verriegelungsposition, geschoben wird. In dieser zweiten Funktionsstellung findet der Spritzenverschluss 1 sicheren Halt am Fortsatz 9 der Spritze 5.

Bei dem hier dargestellten Ausführungsbeispiel ist das mindestens eine Verriegelungselement 27 keilförmig derart ausgebildet, dass es an seinem oberen Ende 37 eine geringere Dicke aufweist als an seinem unteren Ende 39. In radialer Richtung, also senkrecht zur Längsachse 3 gesehen, verdickt sich also das mindestens eine Verriegelungselement 27. Damit ist also die Innenfläche des mindestens einen Verriegelungselements 27 quasi konusförmig ausgebildet: Sie verjüngt sich ausgehend vom oberen Ende 37 in Richtung auf das untere Ende 39.

Durch die bei dem hier dargestellten Ausführungsbeispiel vorgesehene Keilform des mindestens einen Verriegelungselements 27 wird beim Verlagern des Spritzenverschlusses 1 in seine Verriegelungsposition der mit dem Verriegelungselement 27 zusammenwirkende Wandbereich 31 des Spritzenverschlusses 1 in Richtung auf die Längsachse 3 gedrängt, sodass sich durch die axiale Verlagerung des Spritzenverschlusses 1 in Richtung auf den Grundkörper 7 der Spritze 5 ein Formschluss mit der mindestens einen Vertiefung 13 ergibt. Dieser gewährleistet einen sicheren Halt des Spritzenverschlusses 1, der die in Figur 3 wiedergegebene zweite Funktionsstellung einnimmt und sich in seiner Verriegelungsposition befindet.

Bei dem in den Figuren 1 bis 3 dargestellten Ausführungsbeispiel der Spritze 5 ist die mindestens eine Vertiefung 13, die, wie gesagt, vorzugsweise als ringförmige Vertiefung ausgelegt ist, in einem derartigen Abstand zur Schulter 35 des Grundkörpers 7 der Spritze 5 angeordnet, dass in der in Figur 3 wiedergegebenen Position des Spritzenverschlusses 1 und der Verriegelungseinrichtung 25 die Verriegelungseinrichtung 25 so aktiviert ist, dass sie sich in ihrer zweiten Funktionsstellung, der Verriegelungsposition, befindet.

Sollte allerdings die Vertiefung 13 - in Richtung der Längsachse 3 gesehen - einen größeren Abstand zur Schulter 35 aufweisen, so würde die Verriegelungseinrichtung 25 nach dem vollständigen Aufsetzen des Spritzenverschlusses 1 auf den Fortsatz 9 nicht, zumindest nicht so weit wie in Figur 3 dargestellt, in den Spritzenverschluss 1 hineinverlagert. Es müsste dann mittels eines gesonderten Werkzeugs sichergestellt werden, dass nach dem vollständigen Aufsetzen des Spritzenverschlusses 1 auf den Fortsatz 9 die Verriegelungseinrichtung 25 - hier nach oben - verlagert wird, sodass der Wandbereich 31 gegen die Außenfläche der mindestens einen Vertiefung 13 derart angepresst wird, dass ein Formschluss gewährleistet ist.

Besonders bevorzugt wird jedoch das in Figur 3 dargestellte Ausführungsbeispiel, bei dem die Schulter 35 in einem derartigen Abstand zum unteren Ende 41 des Spritzenverschlusses 1 angeordnet ist, dass die Verriegelungseinrichtung 25 derart in den Spritzenverschluss 1 hineingeschoben ist, dass die Verriegelungseinrichtung 25 voll aktiviert ist: Das mindestens eine Verriegelungselement 27 gewährleistet einen Formschluss mit der mindestens einen Vertiefung 13 am Fortsatz 9, hier dadurch, dass der Wandbereich 31 gegen den Boden der mindestens einen Vertiefung 13 angepresst wird.

Figur 4 zeigt eine Unteransicht des Spritzenverschlusses 1. Gleiche und funktionsgleiche Teile sind mit identischen Bezugsziffern versehen, sodass insoweit auf die Beschreibung zu den Figuren 1 bis 3 verwiesen wird.

Figur 4 zeigt, dass das hier dargestellte Ausführungsbeispiel des Spritzenverschlusses 1 nicht nur eine Ausnehmung 29, sondern vier in Umfangsrichtung gesehen vorzugsweise in gleichem Umfangsabstand zueinander angeordnete Ausnehmungen 29 aufweist. Entsprechend ist die Verriegelungseinrichtung 25 so ausgebildet, dass sie vier Verriegelungselemente 27 aufweist, die ebenfalls - in Umfangsrichtung gesehen - in einem gleichen Abstand zueinander angeordnet sind, wobei jeweils ein Verriegelungselement 27 in eine Ausnehmung 29 eingreift.

Figur 4 zeigt, dass die Ausnehmungen 29 nach innen durch einen Wandbereich 31 begrenzt werden. Dabei kann bei mindestens einer Ausnehmung 29, hier bei allen Ausnehmungen, vorgesehen sein, dass der Wandbereich 31 eine Aussparung 43 aufweist, sodass der Wandbereich 31 zwei an die Aussparung 43 angrenzende Teilabschnitte 31a, 31b umfasst, die sich nicht berühren. Ist die Aussparung 43 ganz am Ende eines Wandbereichs 31 angeordnet, so ergibt sich lediglich ein Wandabschnitt.

Wird, wie anhand der Figuren 1 bis 3 erläutert, die Verriegelungseinrichtung 25 aktiviert, wobei das mindestens eine Verriegelungselement 27, gemäß Figur 4 also vier Verriegelungselemente, gegenüber dem Spritzenverschluss 1 verlagert, so wird der Wandbereich 31, beziehungsweise dessen Wandabschnitte 31a, 31b, die an die Aussparung 43 angrenzen, in radialer Richtung nach innen auf die Längsachse 23 ausgelenkt, sodass ein Formschluss des Wandbereichs 31 beziehungsweise der Wandabschnitte 31a, 31b mit der mindestens einen Vertiefung 13 geschaffen wird.

Es ist möglich, dass jedem der vier Wandbereiche 31 beziehungsweise der Wandabschnitte 31a, 31b eine eigene Vertiefung zugeordnet wird. Dann müsste der Spritzenverschluss 1 beim Aufsetzen auf den Fortsatz 9 in eine derartige Drehposition gebracht werden, dass die Wandbereiche 31 zugehörigen Vertiefungen zugeordnet sind. Daher ist vorzugsweise vorgesehen, dass mehreren Wandbereichen 31 beziehungsweise Wandabschnitten 31a, 31b eine gemeinsame Vertiefung zugeordnet wird. Insbesondere wird bevorzugt, dass die Vertiefung 13 ringförmig ausgebildet ist, sodass alle Wandabschnitte 31 in diese ringförmige Vertiefung 13 eingreifen, ohne dass es einer speziellen Drehausrichtung des Spritzenverschlusses 1 gegenüber dem Fortsatz 9 bedürfte.

Figur 5 zeigt in perspektivischer Ansicht von schräg oben ein Ausführungsbeispiel der Verriegelungseinrichtung 25, die mindestens ein, hier vorzugsweise vier Verriegelungselemente 27 aufweist. Die Verriegelungselemente 27 sind durch einen Tragkörper 45 miteinander verbunden, der vorzugsweise alle Verriegelungselemente 27 einer Verriegelungseinrichtung 25 trägt. Er ist insbesondere bevorzugt ringförmig ausgebildet. Dabei ist es also beispielsweise möglich, auch Ringsegmente als Tragkörper vorzusehen, die jeweils einem oder mehreren Verriegelungselementen zugeordnet sind. Besonders einfach handhabbar ist das hier dargestellte Ausführungsbeispiel, bei dem der Tragkörper 45 ringförmig ausgebildet ist und sämtliche Verriegelungselemente 27 erfasst.

Es wird deutlich, dass die Verriegelungseinrichtung 25 auch mehr als vier Verriegelungselemente umfassen könnte, insbesondere dann, wenn der Spritzenverschluss 1 entsprechend ausgebildet und die vorzugsweise identische Anzahl von Ausnehmungen 29 umfasst. Denkbar ist es aber auch, dass eine oder mehrere der Ausnehmungen kein Verriegelungselement aufnimmt, dass also mehr Ausnehmungen vorhanden sind, als Verriegelungselemente vorgesehen werden.

Bei einem abgewandelten Ausführungsbeispiel des Spritzenverschlusses 1 ist vorzugsweise vorgesehen, dass die mindestens eine Ausnehmung 29 im Spritzenverschluss 1 weiches, verformbares, vorzugsweise gelartiges Material aufnimmt, welches als Verriegelungselement dient. Die Verriegelungseinrichtung weist in diesem Fall mindestens einen Verdrängerkörper auf, die bei der Verlagerung der Verriegelungseinrichtung 25, wie sie anhand der Figuren 2 und 3 erläutert wurde, in die mindestens eine Ausnehmung 29 eintreten und das weiche, verformbare Material mit einem Druck beaufschlagen. Derartige Verdrängerkörper sind so angeordnet wie die in den Figuren dargestellten Verriegelungselemente und werden - wie aus den Figuren 1 bis 3 ersichtlich - beim Aufsetzen eines Spritzenverschlusses 1 mehr und mehr in eine zugehörige, mit verformbarem Material gefüllte Ausnehmung eingeschoben. Sie können ohne eine Keilform realisiert werden.

Bei einer ersten Ausführungsform ist der Wandbereich 31 bevorzugt durchgehend ausgebildet und wird durch das weiche, verformbare Material radial nach innen in Richtung auf die mindestens eine Vertiefung 13 ausgelenkt, sodass sich ein Formschluss mit der mindestens einen Vertiefung am Fortsatz ergibt.

Bei einer anderen Ausführungsform ist bevorzugt vorgesehen, dass die mindestens eine Ausnehmung 29 keinen geschlossenen Wandbereich 31, sondern zwei Wandabschnitte 31a, 31b mit einer Aussparung 43 aufweist, durch welche das weiche, verformbare Material bei Eintritt des Verdrängerkörpers in die Aussparung 29 in Richtung auf die mindestens eine Vertiefung 13 verdrängt wird und einen Formschluss zwischen Spritzenverschluss 1 und Fortsatz 9 bewirkt. Das Material dieses weichen Verriegelungselements ist also einerseits verformbar, sodass es bei Eintritt eines Verdrängerkörpers in die Ausnehmung 29 verdrängt wird. Es ist andererseits so formstabil, dass ein sicherer Formschluss zwischen Spritzenverschluss 1 und Grundkörper 7 im Bereich der mindestens einen Vertiefung 13 gewährleistet ist.

Die mindestens eine in den Figuren 1 bis 3 sichtbare Ausnehmung 29 ist bei diesem Ausführungsbeispiel vorzugsweise oben, also im Abstand zum unteren Rand 41 des Spritzenverschlusses 1 verschlossen, sodass das weiches, verformbares Material umfassende Verriegelungselement 27, das auch aus diesem Material bestehen kann, nicht in das Innere des Spritzenverschlusses austreten kann, sondern den Wandbereich 31 in die mindestens eine Vertiefung 13 drängt oder selbst durch eine Aussparung 43 in diese mindestens eine Vertiefung gepresst wird, um in einer zweiten Funktionsstellung die Verriegelungsposition zu gewährleisten, in welcher der Spritzenverschluss 1 durch Formschluss sicher am Fortsatz 9 gehalten wird.

Das anhand der Figuren erläuterte Ausführungsbeispiel des Spritzenverschlusses 1 weist eine Verriegelungseinrichtung 25 mit mindestens einem Verriegelungselement 27 auf, welches von seinem oberen Ende 37 ausgehend eine zunehmende Dicke bis zu seinem unteren Ende 39 aufweist. Damit ergibt sich eine Keilform. Mit anderen Worten, das Verriegelungselement 27 ist als Keil ausgebildet, der bei einer axialen Relativbewegung, also in Richtung der Längsachse 3 gesehen, einen Wandbereich 31 in einer zweiten Funktionsstellung, der Verriegelungsposition, in mindestens eine Vertiefung 13 drängt, um einen Formschluss zu gewährleisten.

Insbesondere aus Figur 4 ist Folgendes ersichtlich:
Der Spritzenverschluss 1 weist mindestens eine Ausnehmung 29 auf, die durch einen Wandbereich 31 nach innen, also in Richtung auf die Längsachse 3 gesehen, die Ausnehmung begrenzt. Denkbar ist es, dass dieser Wandbereich 31 - in Umfangsrichtung gesehen - eine zunehmende Dicke aufweist, sodass sich entsprechend die Ausnehmung 29 - in Umfangsrichtung gesehen - von einem Ende zum anderen Ende verjüngt. Es kann also auch eine Keilform realisiert werden, die in Umfangsrichtung orientiert ist. Wird in eine derartige Ausnehmung mit quasi keilförmigem Wandbereich 31 ein Verriegelungselement eingebracht, welches mit der Innenseite des Wandbereichs 31 zusammenwirkt, so kann in einer ersten Funktionsstellung eine Freigabeposition, und durch eine Relativdrehung des Spritzenverschlusses 1 gegenüber dem Verriegelungselement durch das Zusammenwirken des Verriegelungselements mit dem keilförmigen Wandbereich 31 in einer zweiten Funktionsstellung ein Formschluss mit der mindestens einen Vertiefung 13 am Fortsatz 9 realisiert werden. Der Wandbereich 31 wird bei dieser Ausgestaltung bei einer Drehung des Spritzenverschlusses 1 gegenüber dem Verriegelungselement 27 bei entsprechender Relativdrehung des Wandbereichs 31 gegenüber dem Verriegelungselement 27 nach innen gedrängt, um den Formschluss zu realisieren.

Bei diesem Ausführungsbeispiel ist das Verriegelungselement selbst nicht zwingend keilförmig ausgebildet. Es wirkt vielmehr mit einem - in Umfangsrichtung gesehen - als Keil wirkenden Wandabschnitt 31 zusammen, der in diesem Fall quasi das Verriegelungselement der Verriegelungseinrichtung darstellt.

Der hier beschriebene Spritzenverschluss zeichnet sich also durch eine Verriegelungseinrichtung mit mindestens einem Verriegelungselement aus, welches in einer Funktionsstellung einen Formschluss mit mindestens einer Vertiefung am Fortsatz 9 gewährleistet, sodass der Spritzenverschluss 1 in dieser Position, der Verriegelungsposition, sicher an der Spritze 5 gehalten wird.

Aus den Erläuterungen zum Funktionsprinzip des Spritzenverschlusses 1 wird deutlich, dass dieser mit überaus geringen Kräften über das freie Ende 11 auf den Fortsatz 9 aufgesetzt werden kann. Er weist eine Verriegelungseinrichtung 25 auf, die nach dem Aufsetzen des Spritzenverschlusses 1 aktiviert wird und erst dann den Formschluss mit mindestens einer Vertiefung 13 am Fortsatz 9 der Spritze 5 gewährleistet. Dadurch ist es möglich, eine Materialüberdehnung, wie sie bei Spritzenverschlüssen der herkömmlichen Art üblich ist, beim Aufsetzen des Spritzenverschlusses zu vermeiden. Dies führt einerseits dazu, dass beim Aufsetzen des Spritzenverschlusses 1 auf den Fortsatz 9 relativ geringe Kräfte erforderlich sind, sodass die Gefahr eines Abbrechens des Fortsatzes 9 auf ein absolutes Minimum reduziert ist. Zum anderen wird das Material des Spritzenverschlusses 1 derart geschont, dass es nicht überdehnt wird und daher eine längere Haltbarkeit aufweist; das heißt, auch bei einer relativ langen Lagerungszeit und bei großen Temperaturunterschieden bei der Lagerung oder beim Transport der Spritze wird das Material des Spritzenverschlusses nur minimal beansprucht, sodass Ermüdungserscheinungen und damit ein verschlechterter Sitz des Spritzenverschlusses auf der Spritze vermieden wird.

In vielen Fällen werden Spritzen nach dem Befüllen und dem Aufsetzen des Spritzenverschlusses noch einem letzten Sterilisierprozess unterworfen. Dabei ist auch vorgesehen, dass die Spritze mit dem Spritzenverschluss autoklaviert wird. Hierbei treten hohe Temperaturen von insbesondere über 120°C auf, bei denen ein überdehntes Material des Spritzenverschlusses, wie es beim Stand der Technik gegeben ist, sich so verändert, dass die Haltekräfte des Spritzenverschlusses an der Spritze stark nachlassen. Dies kann zu einem versehentlichen Lösen des Spritzenverschlusses 1 führen.

Anhand von Figur 1 wurde erläutert, dass die Verriegelungseinrichtung 25 mindestens ein Verriegelungselement 27 aufweist, welches bereichsweise in eine Ausnehmung 29 des Spritzenverschlusses 1 eingesteckt ist, sodass sich gemäß den Figuren 1 und 2 eine vormontierte Baueinheit ergibt, bei der der Spritzenverschluss 1 und die Verriegelungseinrichtung 25 gemeinsam gehandhabt werden können. Dabei ist vorzugsweise vorgesehen, dass die Verriegelungseinrichtung 25 im Spritzenverschluss 1 verrastet ist, also über eine Rastverbindung mit dem Spritzenverschluss 1 verbunden ist.

Denkbar ist es aber auch, dass der Spritzenverschluss 1 und die Verriegelungseinrichtung 25 einteilig ausgebildet werden, dass also die Verriegelungseinrichtung 25 Teil des Spritzenverschlusses ist und beispielsweise durch mindestens einen Steg oder einen dünnwandigen Materialbereich mit diesem verbunden ist. Wird eine Kraft auf die Verriegelungseinrichtung 25 ausgeübt, reißen die Stege oder der Verbindungsbereich, also dass eine axiale Verlagerung der Verriegelungseinrichtung 25 in Richtung der Längsachse 3, wie anhand der Figuren 1 bis 3 erläutert, erfolgen kann.

Schließlich ist es möglich, den Spritzenverschluss 1 und die Verriegelungseinrichtung 25 in einem Zweikomponenten-Spritzgussverfahren herzustellen, wobei der Spritzenverschluss 1 und die Verriegelungseinrichtung 25 aus Kunststoffkomponenten bestehen, die sich beim Herstellungsprozess nicht so miteinander verbinden, dass eine Relativbewegung der beiden Elemente gegeneinander verhindert würde.

## Patentansprüche

1. Spritzenverschluss, eingerichtet zum Verschließen einer Spritze (5), die
- einen Grundkörper (7) und
- einen von dem Grundkörper (7) ausgehenden endständigen Fortsatz (9) aufweist, der
- ein freies Ende (11) und eine am Grundkörper (7) angeordnete Basis (15) aufweist, wobei
- an dem Fortsatz (9) in einem Abstand zu dessen freien Ende (11) mindestens eine Vertiefung (13) vorgesehen ist, wobei
- der Spritzenverschluss (1) zum Verschließen der Spritze (5) auf deren Fortsatz (9) über dessen freies Ende (11) derart aufsetzbar ist, dass der Spritzenverschluss (1) die mindestens eine Vertiefung (13) des Fortsatzes (9) übergreift, wobei
- der Spritzenverschluss (1) eine Verriegelungseinrichtung (25) mit
- mindestens einem Verriegelungselement (27) aufweist,
**dadurch gekennzeichnet, dass**
- der Spritzenverschluss (1) auf einer offenen, der Spritze (5) bestimmungsgemäß zugewandten Seite wenigstens eine Ausnehmung (29) aufweist, die eingerichtet ist, um das wenigstens eine Verriegelungselement (27) aufzunehmen, wobei
- die wenigstens eine Ausnehmung (29) radial innenliegend einen verformbaren Wandabschnitt (31) aufweist, wobei
- das wenigstens eine Verriegelungselement (27) in einer ersten Funktionsstellung nach dem Aufsetzen des Spritzenverschlusses (1) auf den Fortsatz (9) eine Freigabeposition einnimmt, und
- nach einer Relativbewegung des Spritzenverschlusses (1) gegenüber der Verriegelungseinrichtung (25) in einer zweiten Funktionsstellung, der Verriegelungsposition, einen Formschluss mit der mindestens einen Vertiefung (13) am Fortsatz (9) bewirkt und damit den Spritzenverschluss (1) verriegelnd am Fortsatz (9) hält, indem
- das mindestens eine Verriegelungselement (27) in der Verriegelungsposition mit dem Wandabschnitt (31) zusammenwirkt und
- den Wandabschnitt (31) in die mindestens eine Vertiefung (13) drängt.

2. Spritzenverschluss nach Anspruch 1, **dadurch gekennzeichnet, dass** die Verriegelungseinrichtung (25) mindestens einen Keil aufweist.

3. Spritzenverschluss nach Anspruch 2, **dadurch gekennzeichnet, dass** der Keil mit dem mindestens einen Verriegelungselement (27) zusammenwirkt und dieses in die Verriegelungsposition verlagert.

4. Spritzenverschluss nach Anspruch 2, **dadurch gekennzeichnet, dass**
- das mindestens eine Verriegelungselement (27) selbst als Keil ausgebildet ist und
- mit Bereichen des Spritzenverschlusses (1) zusammenwirkt.

5. Spritzenverschluss nach Anspruch 1, **dadurch gekennzeichnet, dass** die Verriegelungseinrichtung (25) eine Anzahl von Verriegelungselementen (27) aufweist, die einem Tragkörper (45) zugeordnet sind.

6. Spritzenverschluss nach Anspruch 5, **dadurch gekennzeichnet, dass** der Tragkörper (45) ringförmig ausgebildet ist und die Verriegelungselemente (27) in vorzugsweise gleichem Umfangsabstand zueinander angeordnet sind, wobei jedem der Verriegelungselemente (27) eine Vertiefung zugeordnet ist oder wenigstens einige der Verriegelungselemente (27) in eine gemeinsame Vertiefung eingreifen.

7. Spritzenverschluss nach Anspruch 1, **dadurch gekennzeichnet, dass** der Wandbereich (31) eine Aussparung (43) aufweist.

8. Spritzenverschluss nach Anspruch 1 oder 7, **dadurch gekennzeichnet, dass**
- das mindestens eine Verriegelungselement verformbares Material aufweist oder aus diesem besteht, dass
- die Verriegelungseinrichtung einen Verdrängerkörper aufweist, der
- in der Verriegelungsposition das verformbare Material des Verriegelungselements mit Druck beaufschlagt, sodass
- der mindestens eine verformbare Wandbereich (31) in die mindestens eine Vertiefung (13) drängbar ist, oder sodass
- das verformbare Material in die mindestens eine Vertiefung (13) pressbar ist.

## Claims

1. A syringe closure, configured for closing off a syringe (5) having
- a basic body (7) and
- a terminal extension (9) that extends from the basic body (7),
- the terminal extension (9) having a free end (11) and a base (15) disposed at the basic body (7), wherein
- at least one recess (13) is provided on the terminal extension (9) at a distance from the free end (11), wherein
- the syringe closure (1) can be attached to the extension (9) for closing off the syringe (5) via its free end (11) in such a way that the syringe closure (1) overlaps the at least one recess (13) of the extension (9), wherein
- the syringe closure (1) comprises a locking device (25) having
- at least one locking element (27),
**characterized in that**
- the syringe closure (1) comprises, on an open side facing the syringe (5), at least one recess (29), which is configured to accommodate the at least one locking element (27), wherein
- the at least one recess (29) comprises a radially inwardly located deformable wall area (31), wherein
- the at least one locking element (27) assumes a release position in a first functional position after the attachment of the syringe closure (1) on the extension (9), and
- after a relative movement between the syringe closure (1) and the locking device (25) ensures a positive engagement with the at least one recess (13) on the extension (9) in a second functional position, the locking position, and thus lockingly holds the syringe closure (1) on the extension (9), as
- the at least one locking element (27) interacts with the wall area (31) in the locking position, and
- pushes the wall area (31) into the at least one recess (13).

2. The syringe closure according to claim 1, **characterized in that** the locking device (25) comprises at least one wedge.

3. The syringe closure according to claim 2, **characterized in that** the wedge interacts with the at least one locking element (27) and displaces said at least one locking element into the locking position.

4. The syringe closure according to claim 2, **characterized in that**
- the at least one locking element (27) is configured as a wedge and
- interacts with areas of the syringe closure (1).

5. The syringe closure according to claim 1, **characterized in that** the locking device (25) comprises a number of locking elements (27) which are allocated to one supporting element (45).

6. The syringe closure according to claim 5, **characterized in that** the supporting element (45) is configured in an annular shape and the locking elements (27) are preferably arranged at an identical peripheral distance to each other, wherein a recess is allocated to each of the locking elements (27), or at least some of the locking elements (27) engage into a joint recess.

7. The syringe closure according to claim 1, **characterized in that** wall area (31) has a recess (43).

8. The syringe closure according to claim 1 or 7, **characterized in that**
- the at least one locking element comprises a deformable material or is made therefrom, that
- the locking device has a displacer, which
- applies pressure to the deformable material of the locking element in the locking position, so that
- the at least one deformable wall area (31) can be pushed into the at least one recess (13), or that
- the deformable material can be pressed into the at least one recess (13).

## Revendications

1. Fermeture de seringue configuré pour fermer une seringue (5) comprenant
- un corps de base (7) et
- une saillie (9) terminale partant du corps de base (7), la saillie comprenant
- une extrémité libre (11) et une base (15) située sur le corps de base (7), dans laquelle
- au moins une encoche (13) est prévue au niveau de la saillie (9) dans une distance à son extrémité libre (11),
- la fermeture de seringue (1) pouvant être placé sur la saillie (9) de la seringue (5) sur son extrémité libre (11) pour la fermer, de telle manière que la fermeture de seringue (1) chevauche l'au moins une encoche (13) de la saillie (9),
- la fermeture de seringue (1) comprenant un dispositif de verrouillage (25) avec
- au moins un élément de verrouillage (27),
**caractérisé en ce que**
- la fermeture de seringue (1) présente, sur un côté ouvert qui fait face à la seringue (5) dans la configuration prévue, au moins un évidement (29) configurée pour recevoir l'au moins un élément de verrouillage (27),
- l'au moins un évidement (29) présentant une partie de paroi (31) déformable positionnée radialement à l'intérieur,
- l'au moins un élément de verrouillage (27) occupant, dans une première position fonctionnelle après le placement de la fermeture de seringue (1) sur la saillie (9), une position de déverrouillage, et
- après un mouvement relatif de la fermeture de seringue (1) par rapport au dispositif de verrouillage (25) dans une seconde position fonctionnelle, la position de verrouillage, effectuant un engagement positif avec l'au moins une encoche (13) sur la saillie (9), et ainsi maintenant la fermeture de seringue (1) en verrouillage sur la saillie (9), par
- l'interaction de l'au moins un élément de verrouillage (27) dans la position de verrouillage avec la partie de paroi (31), et en
- poussant la partie de paroi (31) dans l'au moins une encoche (13).

2. Fermeture de seringue selon la revendication 1, **caractérisé en ce que** le dispositif de verrouillage (25) présente au moins une cale.

3. Fermeture de seringue selon la revendication 2, **caractérisé en ce que** la cale coopère avec l'au moins un élément de verrouillage (27) et le déplace vers la position de verrouillage.

4. Fermeture de seringue selon la revendication 2, **caractérisé en ce que**
- l'au moins un élément de verrouillage (27) lui-même est configuré sous forme de cale et
- coopère avec des régions de la fermeture de seringue (1).

5. Fermeture de seringue selon la revendication 1, **caractérisé en ce que** le dispositif de verrouillage (25) présente un nombre des éléments de verrouillage (27) qui sont associés à un corps de support (45).

6. Fermeture de seringue selon la revendication 5, **caractérisé en ce que** le corps de support (45) est configuré sous forme annulaire, et les éléments de verrouillage (27) sont arrangés dans une distance circonférentielle entre eux qui est de préférence égale, chacun des éléments de verrouillage (27) étant associé à une encoche respectif, ou au moins certains des éléments de verrouillage (27) venant en prise avec une encoche commun.

7. Fermeture de seringue selon la revendication 1, **caractérisé en ce que** la région de paroi (31) présente une cavité (43).

8. Fermeture de seringue selon la revendication 1 ou 7, **caractérisé en ce que**
- l'au moins un élément de verrouillage présente ou consiste d'un matériau déformable, **en ce que**
- le dispositif de verrouillage présente un corps déplaceur qui,
- en position de verrouillage, applique une pression sur le matériau déformable de l'élément de verrouillage, de telle manière que
- l'au moins une région de paroi (31) déformable peut être poussée ladite au moins une encoche (13), ou de telle manière que
- le matériau déformable peut être pressé dans ladite au moins une encoche (13).
